# DEMANDE DE BREVET EUROPEEN

(11) **EP 1 982 744 A1**
(43) Date de publication de la demande: **22.10.2008**
(21) Numéro de dépôt: 08300186.7
(22) Date de dépôt: 18.04.2008
(51) Int. Cl.: A61M 39/02, A61M 39/04

(54) **Septum de chambre implantable et chambre implantable le comportant**

(30) Priorité: 20.04.2007 FR 0702897
(71) Demandeur: B.Braun Medical SAS, 92100 Boulogne-Billancourt (FR)
(72) Inventeur: Rimbot, Reynald, 86130, SAINT CYR (FR)
(74) Mandataire: Lebrette, Camille

(57) **Abrégé**

L'invention concerne un septum de chambre implantable.

Elle se rapporte à un septum (26) destiné à être perforé par des aiguilles creuses et comprenant une partie de maintien et une partie centrale dont les surfaces sont dégagées pour pouvoir être perforées par des aiguilles. Le septum (26) a un état de travail dans lequel des éléments périphériques exercent sur lui des forces de compression dans la direction de son épaisseur telles que le septum (26) subit un gonflement en direction perpendiculaire à son plan lorsqu'il passe de sa position de repos à sa position de travail. A l'état de repos, les surfaces de la partie centrale forment des cavités (28) pratiquement sur toute leur étendue.

Application aux chambres implantables.

## Description

L'invention concerne un septum pour chambre implantable, et une chambre implantable le comportant.

On utilise des chambres implantables pour l'introduction et/ou l'extraction de fluides d'un corps humain. Comme la chambre est totalement implantée dans le corps, l'accès à celle-ci est obtenu avec une aiguille qui pénètre dans la peau du patient et traverse le septum pour établir ainsi la communication entre l'extérieur du corps et le volume intérieur de la chambre.

De telles aiguilles ont un effet de "carottage", c'est-à-dire un effet de découpe d'un cylindre du matériau du septum.

Pour éviter ce phénomène de carottage, on sait déjà utiliser certaines aiguilles adaptées. Par exemple, les aiguilles de Huber ont un biseau tangentiel associé à une courbure de l'aiguille au niveau de la pointe, afin que le biseau soit pratiquement orienté dans la direction d'introduction.

Dans certaines applications des chambres implantables, par exemple à la chimiothérapie, un faible débit de liquide dans l'aiguille suffit, de sorte que des aiguilles de petite dimension, de numéro 19G ou 20G, suffisent. Des aiguilles aussi minces ne provoquent qu'une perturbation modérée de la matière du septum, si bien qu'il est possible d'utiliser une telle chambre un nombre de fois bien supérieur au nombre de perforations nécessaire en réalité avant que le septum ne soit détérioré.

Cependant, dans d'autres applications, notamment l'hémodialyse, il est nécessaire de faire circuler un débit relativement élevé dans la chambre, et on utilise des aiguilles plus grosses, par exemple de numéro 14G à 17G. De telles aiguilles, même adaptées, par exemple de type de Huber, ont un effet important d'endommagement du septum.

On connaît aussi des aiguilles à mandrin dont la lumière est fermée, pendant l'insertion, par un mandrin ayant une dimension correspondant au diamètre de la lumière afin que l'aiguille ne donne pas de carottage. Les extrémités de l'aiguille et du mandrin sont taillées en pointe. Lorsque l'aiguille a traversé le septum, le mandrin est retiré et le corps du dispositif d'injection (seringue, circuit d'hémodialyse, etc.) est raccordé à l'aiguille.

Cependant, comme une hémodialyse doit être réalisée fréquemment sur les patients concernés, c'est-à-dire plusieurs fois par semaine, et comme le traitement par hémodialyse peut durer des années, le nombre de perforations du septum est très élevé. Il pourrait même arriver que la détérioration du septum par carottage impose le remplacement de la chambre implantable.

L'invention a pour objet la solution du problème précité, et elle permet d'éviter ou de réduire la détérioration du septum par les aiguilles, surtout de gros diamètre.

Plus précisément, l'invention a pour objet un septum ayant des propriétés telles qu'il permettrait l'utilisation d'une même chambre implantable pendant plusieurs années dans le cas de l'hémodialyse.

Dans le procédé connu de fabrication des chambres implantables, un flan destiné à former un septum est obtenu par moulage. Ce flan, logé dans un alvéole qui maintient sa périphérie, est alors serti entre deux éléments périphériques ou couronnes rigides qui sont rapprochés dans la direction de l'épaisseur du septum. Le rapprochement provoque la compression du matériau, de sorte que celui-ci est repoussé vers le centre du septum et que le septum est bombé à chacune de ses grandes faces entre les couronnes de compression.

Selon que la force de compression exercée par les éléments périphériques à la partie marginale des grandes faces du flan est plus ou moins élevée, les bombements du matériau du septum dus aux contraintes internes sont plus ou moins importants. Une compression élevée augmente le nombre de perforations qu'il est possible de réaliser, mais surtout dans la partie périphérique du septum.

L'invention permet une augmentation considérable du nombre de perforations qui peut être obtenu pour un même volume apparent du septum et pour des contraintes de valeurs semblables, permettant des forces de pénétration d'aiguille acceptables.

Selon l'invention, la résistance à la détérioration du septum est augmentée par application au septum, qui peut être de forme circulaire, elliptique ou autre, de contraintes maîtrisées telles que d'une part le septum résiste à un plus grand nombre de perforations par unité de surface, et d'autre part la zone favorable du septum dans laquelle les perforations peuvent être effectuées est agrandie par rapport à la technique classique, pour une même dimension de septum.

Selon l'invention, ce résultat est obtenu parce que l'une des surfaces au moins du septum forme une cavité lorsqu'il est à l'état de repos, c'est-à-dire hors contraintes.

Bien que l'invention ne soit nullement limitée par une théorie quelconque d'interprétation des résultats obtenus, on peut observer que la présence d'une cavité à l'état de repos permet, à l'état de travail obtenu par compression, la formation d'une surface plane ou peu bombée telle que dans le volume du septum les contraintes varient peu. Le septum selon l'invention présente un volume favorable à la ponction plus étendu que dans le cas d'un septum classique.

Plus précisément, l'invention concerne un septum de chambre implantable destiné à être perforé par des aiguilles creuses, le septum étant disposé globalement suivant un plan sensiblement perpendiculaire à la direction des aiguilles de perforation et comprenant une partie de maintien, disposée sur une portion au moins de sa périphérie et destinée à être serrée par des éléments périphériques, et une partie centrale, dont les surfaces sont dégagées pour pouvoir être perforées par des aiguilles, le septum ayant un état de repos et un état de travail, tels qu'à l'état de repos, l'une au moins des surfaces de la partie centrale forme une cavité pratiquement sur toute son étendue et qu'à l'état de travail les éléments périphériques de compression exercent sur le septum des forces de compression sensiblement perpendiculaires audit plan, de sorte que la partie centrale du septum subit un gonflement en direction perpendiculaire audit plan lorsqu'elle passe de sa position de repos à sa position de travail, de manière à ce que la partie centrale du septum présente un volume dans lequel les contraintes varient peu.

De préférence, la profondeur de la cavité comprise entre la surface de la partie centrale et le plan correspondant de la surface de la partie de maintien présente une variation progressive de la périphérie vers le centre.

De préférence, la profondeur de la cavité comprise entre la surface de la partie centrale et le plan correspondant de la surface de la partie de maintien est pratiquement constante sur le quart environ de son aire autour du centre de la surface.

De préférence, la profondeur de la cavité est telle que, à l'état de travail, la surface correspondante du septum comprimé prend une forme pratiquement plane.

Dans un mode de réalisation, le septum présente une cavité à chacune de ses faces.

Dans un autre mode de réalisation, l'autre surface de la partie centrale n'a pas de cavité de sorte que, à l'état de travail, elle prend une forme bombée.

Dans un autre mode de réalisation, l'autre surface de la partie centrale, à l'état de repos du septum, a une forme bombée.

De préférence, le septum est constitué d'un élastomère de silicone.

L'invention concerne aussi une chambre implantable, du type qui comprend un corps délimitant un volume ayant une ouverture fermée par un septum et un canal de sortie ; selon l'invention, le septum est selon les paragraphes précédents, et la chambre possède des éléments périphériques de compression qui coopèrent avec la partie de maintien du septum et avec l'ouverture du corps.

De préférence, les éléments périphériques de compression délimitent une ouverture circulaire qui dégage les surfaces centrales du septum.

De préférence, les éléments périphériques de compression délimitent une ouverture allongée ayant deux côtés sensiblement parallèles.

Dans une variante, les éléments périphériques de compression exercent aussi une force de compression du septum de la périphérie vers le centre, de sorte que du matériau du septum est déplacé vers le centre.

Le principal avantage de l'invention est l'augmentation du nombre de perforations réalisables dans des conditions de sécurité, de sorte que l'invention permet d'obtenir le nombre de ponctions nécessaire pour des applications telles que l'hémodialyse. En pratique, cela signifie que le septum permet un nombre suffisant de ponctions sans détérioration excessive et sans nécessiter une augmentation conséquente de la force qui doit être appliquée à l'aiguille pour sa pénétration. Ainsi, les utilisateurs, le plus souvent des infirmières, n'ont pas à modifier leur manière de ponctionner.

D'autres caractéristiques et avantages de l'invention seront mieux compris à la lecture de la description qui va suivre d'exemples de réalisation, faite en référence aux dessins annexés sur lesquels :
la figure 1 est une coupe d'une chambre implantable classique ;
les figures 2A, 2B et 2C sont des coupes centrales d'un quart du matériau d'un septum classique destiné à la chambre de la figure 1, représentant les courbes de niveaux de contraintes respectivement en direction radiale, en direction perpendiculaire au rayon mais dans le plan du septum, et en direction perpendiculaire au plan du septum ;
la figure 3 une coupe transversale centrale d'un septum à cavités symétriques selon l'invention ;
les figures 4A, 4B et 4C sont des coupes centrales d'un quart du matériau du septum de la figure 3, représentant les courbes de niveaux de contraintes respectivement en direction radiale, en direction perpendiculaire au rayon mais dans le plan du septum, et en direction perpendiculaire au plan du septum ;
la figure 5 une coupe transversale centrale d'un septum à cavité sur une face et bombement sur l'autre face selon l'invention ;
les figures 6A, 6B et 6C sont des coupes d'une moitié du matériau du septum de la figure 5, représentant les courbes de niveaux de contraintes respectivement en direction radiale, en direction perpendiculaire au rayon mais dans le plan du septum, et en direction perpendiculaire au plan du septum ; et
la figure 7 est une vue schématique en perspective illustrant l'invention dans le cas d'un septum de forme allongée présentant une cavité à bords parallèles.

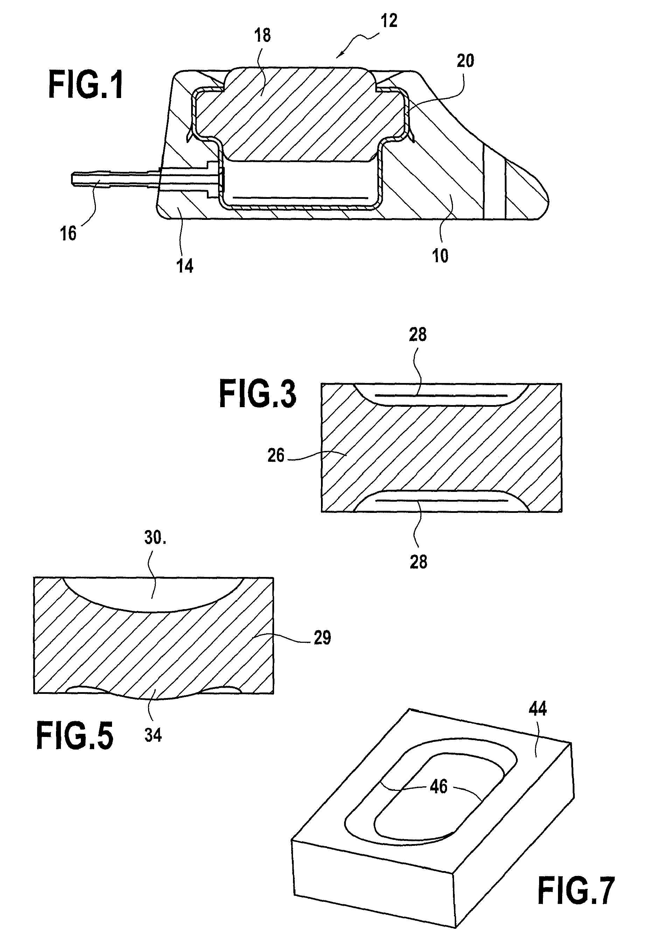

La figure 1 représente une coupe d'une chambre implantable ayant un corps 10 qui délimite un volume compris entre une ouverture 12, formée à sa partie supérieure, et un canal 14, formé à sa partie inférieure et destiné au passage d'une canule 16 de sortie de la chambre. L'ouverture 12 est fermée par un septum 18 maintenu à sa périphérie par des éléments 20 de compression. Ces derniers exercent une compression dans la direction de l'épaisseur du septum. Dans un perfectionnement, la compression peut aussi avoir une composante latérale.

Le corps 10 est habituellement formé de matière plastique moulée, et les éléments périphériques de compression 20, dont l'un forme avantageusement toute la paroi du volume intérieur, sont avantageusement métalliques. Cependant, il est aussi possible que tous les éléments de la chambre soient formés de matière plastique.

La figure 1 représente la forme bombée du septum 18 à ses deux surfaces principales, obtenue par compression d'un flan à faces parallèles.

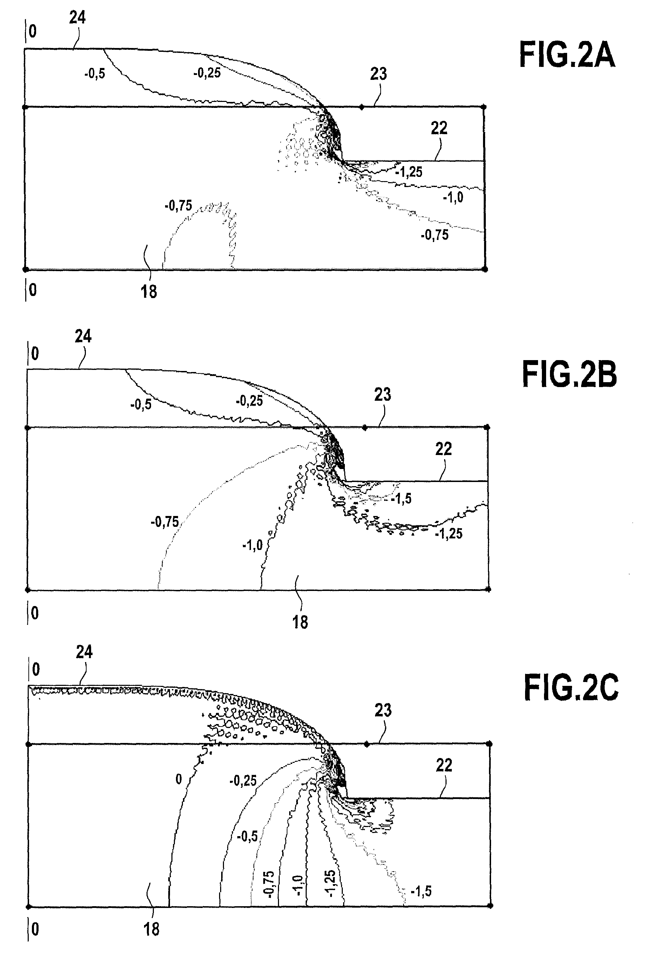

Les figures 2A, 2B et 2C sont des coupes illustrant la répartition des contraintes dans un quart d'un tel septum à l'état de travail. L'axe O-O est l'axe central du septum qui est circulaire, et le rectangle 23 indique la forme de la partie concernée du septum à l'état de repos, avant compression. Les nombres adjacents aux lignes de niveaux indiquent la valeur des contraintes, dans la direction concernée, sur une échelle arbitraire.

Le septum 18 comprend une partie de maintien 22 placée à sa périphérie, et une partie centrale 24. Lorsque les éléments périphériques de compression 20 réduisent l'épaisseur du flan, la partie centrale 24 du septum 18 forme deux bombements aux deux faces opposées.

On note sur les figures 2A et 2B, qui représentent les lignes de niveaux de contraintes en direction radiale (direction horizontale dans le plan de la figure 2A) et en direction perpendiculaire au rayon mais dans le plan du septum (direction horizontale perpendiculaire au plan de la figure 2B), que ces contraintes agissant dans le plan du septum varient beaucoup.

L'invention a pour effet de supprimer ou de réduire ces variations des contraintes dans le volume du septum par maîtrise du bombement de la partie centrale 24. Cette maîtrise est obtenue par utilisation d'un flan de forme adaptée.

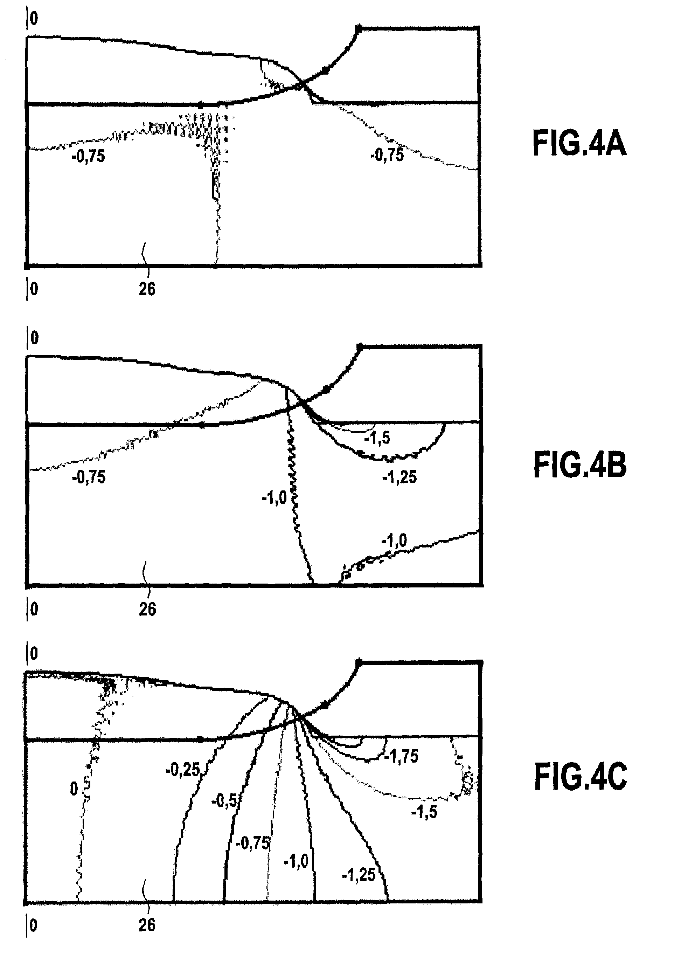

Dans un premier mode de réalisation illustré par les figures 3 et 4A, 4B et 4C, le septum 26 comporte une partie périphérique de maintien, et chacune des deux surfaces opposées a une cavité 28. De préférence, la distance comprise entre le fond de la cavité et le plan délimité par la surface de la partie de maintien, c'est-à-dire la profondeur de la cavité, augmente progressivement, bien que la partie centrale puisse avoir une profondeur sensiblement constante sur le quart au moins de l'aire totale de la cavité.

Dans ce mode de réalisation de la figure 3, les bombements formés aux deux surfaces sont moins importants que dans le cas d'une chambre classique, comme l'indique la comparaison des figures 4A et 4B aux figures 2A et 2B, les contraintes agissant dans le plan du septum varient beaucoup moins dans le volume dans le cas du septum de la figure 3 que dans celui de la figure 1. La répartition de contraintes dans le volume du septum des figures 4A et 4B est beaucoup plus régulière que celle des figures 2A et 2B.

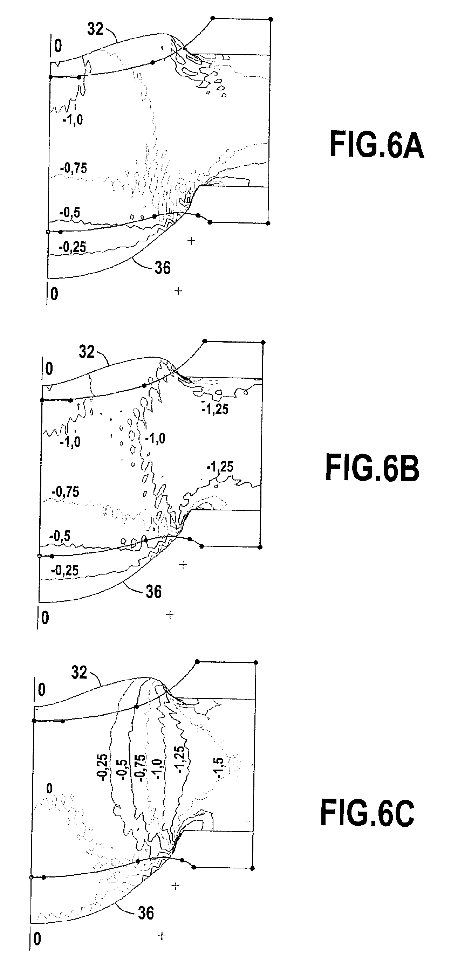

Les figures 5 et 6A, 6B et 6C représentent un autre mode de réalisation de septum 29 dans lequel, à l'état de repos, la cavité 30 d'une surface est plus importante que celle de la figure 3, de sorte que, comme l'indique les figures 6A, 6B et 6C, à l'état comprimé de travail, la surface 32 présente à l'extérieur de la chambre une forme globalement plane, qui est légèrement en saillie et légèrement en creux.

A l'autre face, le flan a une saillie centrale 34 qui donne un bombement accru 36 du côté destiné à être à l'intérieur de la chambre implantable. Sur la figure 6C, les faces supérieure et inférieure présentent une répartition régulière de contraintes.

La figure 7 représente un exemple de cavité non circulaire, de forme allongée. En effet, la chambre peut avoir une ouverture allongée ayant deux bords sensiblement parallèles. Dans ce cas, la cavité réalisée selon l'invention dans un flan 44 a une forme allongée ayant deux côtés 46 qui sont parallèles aux côtés du flan. Dans ce mode de réalisation, la surface de perforation peut être agrandie à volonté, dans les limites de dimensions d'une chambre qui peut être raisonnablement implantée, afin que le nombre de perforations réalisables puisse augmenter. Dans ce cas, qui n'est pas limité aux formes allongées, en plus d'une compression perpendiculaire au plan du flan, il est possible d'ajouter une compression destinée à rapprocher l'un de l'autre les deux bords longitudinaux.

En plus des indications précédentes relatives aux figures 2A, 2B et 2C, 4A, 4B et 4C, et 6A, 6B et 6C, la comparaison des figures 2C, 4C et 6C permet d'observer des répartitions analogues des lignes de niveaux de contraintes dans la direction de perforation. Le réaction du septum à une perforation est donc sensiblement la même pour l'infirmière, qu'il s'agisse d'un septum classique ou d'un septum selon l'invention.

Par contre la comparaison des figures 2A, 2B aux figures 4A, 4B et 6A, 6B respectivement les contraintes agissant dans le volume du septum varient beaucoup moins dans le cas des septums selon l'invention que dans celui du septum classique, c'est-à-dire que la répartition de contraintes dans le volume des septums selon l'invention est beaucoup plus régulière que celle du septum classique.

### Essais démontrant l'efficacité de l'invention

Des essais de perforation de septums dans les conditions d'utilisation ont été réalisés pour démontrer l'effet de l'invention.

Au cours de ces essais, on a comparé divers septums selon l'invention à un septum classique O, disponible dans le commerce dans les chambres par exemple de type "Celsite ST301 ", disponibles auprès de B.Braun Medical.

Un premier septum A à deux cavités, correspondant à la figure 3, était identique au septum classique O, au point de vue des dimensions et du matériau, mis à part la présence des cavités qui laissaient une épaisseur de 6 mm au centre.

Un second septum B à valve, correspondant à la figure 5, était identique au septum classique O, au point de vue des dimensions et du matériau, mis à part la cavité de 4 mm de profondeur et la saillie de l'autre face.

Un troisième septum C était simplement de forme rectangulaire, sans présence de cavités sur ses faces, et il était soumis à la fois à une compression perpendiculaire à son plan et à une compression dans la direction de son plan.

Tous les septums O, A, B et C avaient en position de travail des épaisseurs similaires d'environ 8 mm.

Le tableau qui suit indique le nombre de perforations réalisées au cours d'un essai effectué avec une même aiguille "Surecan" 19G pour chaque septum. Cinq septums de chaque type ont été soumis aux essais, et ont donné une plage de résultats et une valeur moyenne. Les essais ont été réalisés d'une part en ponctionnant des cercles de surface équivalant au cinquième de la surface apparente, ces cercles étant soit centrés, soit excentrés, et d'autre part en ponctionnant la totalité de la surface.

| Ponctions | Septum O | Septum A | Septum B | Septum C |
|---|---|---|---|---|
| Centrée | | | | |
| Minimum | 320 | 1250 | >2000 | 1300 |
| Maximum | 1000 | 1750 | >2000 | 2000 |
| Moyenne | 708 | 1530 | >2000 | 1570 |
| Excentrée | | | | |
| Minimum | 740 | 1150 | 1000 | 700 |
| Maximum | 1350 | 1350 | 1600 | 1650 |
| Moyenne | 1084 | 1280 | 1290 | 1250 |
| Sur totalité | | | | |
| Minimum | 60 | >2000 | >2000 | >2000 |
| Maximum | 1350 | >2000 | >2000 | >2000 |
| Moyenne | 676 | >2000 | >2000 | >2000 |

Une première constatation est le fait que, avec un septum classique, le nombre de perforations obtenu en position excentrée est plus grand qu'en position centrée. Au contraire, avec les trois septums A, B et C selon l'invention, ce nombre est au contraire plus grand en position centrée qu'en position excentrée. Ce résultat est très important car, pour des raisons évidentes de sécurité, les infirmières préfèrent introduire l'aiguille au centre de la surface du septum que sur les bords, pour éviter de piquer au-delà du bord de la chambre.

De plus, les trois types de septum selon l'invention sont nettement supérieurs au septum classique sur le plan du nombre de perforations qui dépasse 2 000 perforations, valeur à laquelle l'essai a été interrompu car elle est supérieure au nombre de perforations pouvant être effectuées sur un patient réel. Au contraire, le septum connu n'atteint qu'une valeur de l'ordre de 700 en moyenne.

Bien entendu, si l'essai était effectué avec des aiguilles plus grosses (14G à 17G), les nombres obtenus seraient plus petits.

## Revendications

1. Septum de chambre implantable destiné à être perforé par des aiguilles creuses, le septum (26, 29, 44) étant disposé globalement suivant un plan sensiblement perpendiculaire à la direction des aiguilles de perforation et comprenant une partie de maintien, disposée sur une portion au moins de sa périphérie et destinée à être serrée par des éléments périphériques, et une partie centrale, dont les surfaces sont dégagées pour pouvoir être perforées par des aiguilles, le septum (26, 29, 44) ayant un état de repos et un état de travail, **caractérisé en ce qu'**à l'état de repos, l'une au moins des surfaces de la partie centrale forme une cavité (28, 30) pratiquement sur toute son étendue et qu'à l'état de travail les éléments périphériques de compression exercent sur le septum des forces de compression sensiblement perpendiculaires audit plan, de sorte que la partie centrale du septum (26, 29, 44) subit un gonflement en direction perpendiculaire audit plan lorsqu'elle passe de sa position de repos à sa position de travail, de manière à ce que la partie centrale du septum présente un volume dans laquelle les contraintes varient peu.

2. Septum selon la revendication 1, **caractérisé en ce que** la profondeur de la cavité (28, 30) comprise entre la surface de la partie centrale et le plan correspondant de la surface de la partie de maintien présente une variation progressive de la périphérie vers le centre.

3. Septum selon l'une des revendications 1 et 2, **caractérisé en ce que** la profondeur de la cavité (28, 30) comprise entre la surface de la partie centrale et le plan correspondant de la surface de la partie de maintien est pratiquement constante sur le quart environ de son aire autour du centre de la surface.

4. Septum selon l'une quelconque des revendications 1 à 3, **caractérisé en ce que** la profondeur de la cavité (28, 30) est telle que, à l'état de travail, la surface correspondante du septum comprimé prend une forme pratiquement plane.

5. Septum selon l'une quelconque des revendications 1 à 4, **caractérisé en ce que** l'autre surface de la partie centrale n'a pas de cavité de sorte que, à l'état de travail, elle prend une forme bombée.

6. Septum selon la revendication 5, **caractérisé en ce que** l'autre surface de la partie centrale, à l'état de repos du septum, a une forme bombée.

7. Chambre implantable, du type qui comprend un corps délimitant un volume ayant une ouverture fermée par un septum (26, 29, 44) et un canal de sortie, **caractérisée en ce que** son septum (26, 29, 44) est selon l'une quelconque des revendications précédentes, et la chambre possède des éléments périphériques de compression qui coopèrent avec la partie de maintien du septum (26, 29, 44) et avec l'ouverture du corps.

8. Chambre implantable selon la revendication 7, **caractérisée en ce que** les éléments périphériques de compression délimitent une ouverture circulaire qui dégage les surfaces centrales du septum (26, 29, 44).

9. Chambre implantable selon la revendication 7, **caractérisée en ce que** les éléments périphériques de compression délimitent une ouverture allongée ayant deux côtés sensiblement parallèles.

10. Chambre implantable selon l'une quelconque des revendications 7 à 9, **caractérisée en ce que** les éléments périphériques de compression exercent aussi une force de compression du septum de la périphérie vers le centre.
